# FASCICULE DE BREVET EUROPEEN

(11) **EP 4 594 740 B1**
(45) Date de publication et mention de la délivrance du brevet: **27.05.2026**
(21) Numéro de dépôt: 23793914.5
(22) Date de dépôt: 28.09.2023
(51) Int. Cl.: G01N 23/221, G01V 5/00

(54) **PROCÉDÉ POUR EXTRAIRE DES COMPOSANTES NEUTRONIQUES DISCRÈTES ISSUES DE RÉACTIONS PHOTO-NUCLÉAIRES À L'AIDE D'UN RÉSEAU DE NEURONES ENTRAINÉ**
VERFAHREN ZUR EXTRAKTION DISKRETER NEUTRONENKOMPONENTEN AUS PHOTONUKLEAREN REAKTIONEN UNTER VERWENDUNG EINES TRAINIERTEN NEURONALEN NETZWERKS
METHOD FOR EXTRACTING DISCRETE NEUTRON COMPONENTS DERIVED FROM PHOTONUCLEAR REACTIONS USING A TRAINED NEURAL NETWORK

(30) Priorité: 30.09.2022 FR 2210022
(43) Date de publication de la demande: 06.08.2025
(73) Titulaire: Commissariat à l'Energie Atomique et aux Energies Alternatives, 75015 Paris (FR)
(72) Inventeur: BESNARD VAUTERIN, Clément, 91191 Gif-sur-Yvette Cedex (FR); BLIDEANU, Valentin, 91191 Gif-sur-Yvette Cedex (FR); RAPP, Benjamin, 91191 Gif-sur-Yvette Cedex (FR)
(74) Mandataire: Santarelli
(86) Numéro de dépôt international: PCT/FR2023/051502
(87) Numéro de publication internationale: WO 2024/069107

(56) Documents cités:
- MCFEE J.E. ET AL: "Photoneutron spectroscopy using monoenergetic gamma rays for bulk explosives detection", NUCLEAR INSTRUMENTS & METHODS IN PHYSICS RESEARCH. SECTION A, vol. 704, 1 March 2013 (2013-03-01), NL, pages 131 - 139, XP093047824, ISSN: 0168-9002, DOI: 10.1016/j.nima.2012.12.053
- SOHRABI MEHDI ET AL: "Photoneutron spectrometry by novel multi-directional spherical neutron spectrometry system", vol. 11, no. 1, 5 February 2021 (2021-02-05), XP093047809, Retrieved from the Internet <URL:https://www.nature.com/articles/s41598-021-81529-5.pdf> DOI: 10.1038/s41598-021-81529-5
- JINIA ABBAS J ET AL: "An Artificial Neural Network System for Photon-Based Active Interrogation Applications", IEEE ACCESS, IEEE, USA, vol. 9, 27 August 2021 (2021-08-27), pages 119871 - 119880, XP011875947, DOI: 10.1109/ACCESS.2021.3108406
- SEGEBADE CHRISTIAN ET AL: "Principles, methodologies, and applications of photon activation analysis: a review", JOURNAL OF RADIOANALYTICAL AND NUCLEAR CHEMISTRY, AKADEMIAI KIADO RT, HU, vol. 312, no. 3, 4 April 2017 (2017-04-04), pages 443 - 459, XP036246416, ISSN: 0236-5731, [retrieved on 20170404], DOI: 10.1007/S10967-017-5238-6

## Description

### DOMAINE TECHNIQUE

Le domaine technique de l'invention est celui de l'exploitation de spectres neutroniques à l'aide d'un réseau de neurones pour en retirer des informations utiles, par exemple dans le cadre de la détection de substances dangereuses, toxiques ou illicites contenues dans un objet.

### ÉTAT DE LA TECHNIQUE ANTÉRIEURE

Il est connu que les sources de rayonnement neutron et photon peuvent être utilisées pour détecter, à partir de réactions nucléaires spécifiques, la présence de certains éléments légers (à savoir l'azote, le carbone, l'oxygène, le chlore) dans divers matériaux. On peut ainsi détecter la présence de substances dangereuses, toxiques ou illicites telles que des explosifs, certains gaz toxiques (gaz toxiques contenant du chlore) ou des stupéfiants, contenues ou dissimulées dans un objet à sonder (qui peut, par exemple, être de type containeur, colis ou valise).

Parmi les méthodes de détection connues, on peut citer la méthode d'interrogation neutronique active (INA), la méthode dite du photon « taggé » et la méthode d'interrogation photonique active (IPA).

La méthode d'interrogation neutronique active (INA) utilise les réactions induites par des neutrons. Ces réactions sont utilisées pour détecter des éléments légers (C, N, O, CI) dans des matériaux en utilisant notamment la technique de la particule associée (TPA). Cette détection est basée sur la mesure du rayonnement gamma produit par ces réactions. Cependant, la mesure précise des spectres gamma est en général difficile compte tenu des conditions expérimentales, souvent caractérisées par un bruit de fond important. En effet, le rayonnement gamma est largement présent, car il peut être facilement produit par une multitude de mécanismes (réactions avec d'autres éléments chimiques que les éléments chimiques d'intérêt, décroissance radioactive des radio-isotopes créés par activation ou qui sont naturellement présents dans les matériaux, etc.), ce qui conduit à des spectres complexes rendant difficile la séparation des différentes contributions des éléments chimiques.

La technique dite du photon « taggé » est basée sur la détection des neutrons en coïncidence avec les électrons d'une source de rayonnement bremsstrahlung. Cette technique permet l'extraction des composantes neutroniques pour chaque énergie des photons de bremsstrahlung. Mais elle nécessite la mise en place de méthodes complexes avec une électronique spécialisée haute fréquence pour la détection de particules en coïncidence, ainsi que des équipements lourds, contraignants et onéreux, notamment des dipôles magnétiques qui ne sont pas vraiment compatibles avec le développement d'un dispositif de détection compact dans le cas d'une application en sécurité intérieure.

Enfin, la méthode d'interrogation photonique active (IPA) est une détection basée sur l'utilisation de sources de photons. Elle utilise les réactions photo-nucléaires dont les sections efficaces (probabilités) sont bien connues pour la plupart des noyaux des éléments légers tels que l'azote, le carbone, l'oxygène, le chlore.

Les réactions photo-nucléaires ont la particularité d'être réalisées à seuil, c'est-à-dire qu'elles ne peuvent avoir lieu que si les photons initiaux ont une énergie supérieure à une certaine valeur, qui est spécifique à chaque noyau.

Dans le cas de l'azote, par exemple, qui est détecté à partir des neutrons produits par des réactions photo-nucléaires (y,n), l'énergie minimale des photons doit être supérieure à 10 MeV.

Dans le cas de la détection du chlore par irradiation avec des photons par la méthode IPA, les techniques actuelles sont basées sur la création, par des réactions photo-nucléaires (y,n), de radio-isotopes dont la décroissance est à l'origine d'émission de rayonnement gamma. La mesure par spectrométrie de ce rayonnement gamma permet d'identifier ces radio-isotopes et de remonter ainsi aux éléments à l'origine de leur production. Cependant, la méthode d'IPA couplée à de la spectrométrie gamma est très complexe à mettre en place étant donné l'importance du bruit gamma généré par de nombreux mécanismes.

Les photons utilisés dans l'interrogation photonique active ne peuvent donc pas être obtenus avec des méthodes conventionnelles basées sur des sources radioactives, car elles ne permettent pas d'atteindre des niveaux d'énergies suffisants. Il est donc nécessaire de disposer de sources de photons de haute énergie suffisamment intenses pour obtenir un rapport signal/bruit satisfaisant. Par ailleurs, la source doit en outre être la plus compacte possible pour être déployée sur le terrain, dans le cas par exemple d'une application à la détection de matières illicites.

Traditionnellement, les photons de haute énergie (supérieure ou égale à 6 MeV) utilisés pour mettre en œuvre la méthode IPA sont des photons de bremsstrahlung, qui sont produits à partir d'un accélérateur d'électrons. De manière connue, dans un accélérateur d'électrons, un faisceau d'électrons d'énergie suffisante (plusieurs MeV) frappe une cible constituée d'un matériau de numéro atomique Z élevé, ce qui donne lieu au rayonnement de freinage (photons de bremsstrahlung). Le désavantage d'utiliser un accélérateur d'électrons réside dans le fait que la source de photons ainsi obtenue (photons de bremsstrahlung) est caractérisée par un spectre en énergie qui est continu. Or, seulement quelques pourcents des photons obtenus par cette méthode (i.e. ceux ayant une énergie supérieure à 6 MeV nécessaire pour induire des réactions de photofission (y,f)) peuvent être utilisés, la grande majorité des photons étant à l'origine d'une irradiation inutile des substances présentes dans l'objet à sonder.

Idéalement, une telle source devrait être basée sur l'utilisation de photons mono-énergétiques obtenus, par exemple, à partir d'une source de protons de basse énergie, afin de pouvoir identifier des signatures spécifiques (ou empreintes) composées de composantes neutroniques discrètes) dans les spectres des neutrons émis dans des réactions (γ,n) induites par ces photons sur des noyaux légers (C, N, O, Cl). Des composantes neutroniques discrètes seront la signature de la présence de certains éléments légers ; la position de ces composantes neutroniques discrètes nous renseignera sur la nature des éléments légers en question et la hauteur de ces composantes neutroniques discrètes nous renseignera sur leur quantité relative.

Un exemple d'interrogation photonique active est décrit dans MCFEE J.E. ET AL: "Photoneutron spectroscopy using monoenergetic gamma rays for bulk explosives detection",NUCLEAR INSTRUMENTS & METHODS IN PHYSICS RESEARCH (2013).

Cependant, de précédents travaux des inventeurs visant à étudier la mise en place d'une source à photons mono-énergétiques ont montré de nombreuses limitations liées, notamment, à l'intensité que pourrait avoir une telle source et à son isotropie.

Dans le cadre de la présente invention, les inventeurs se sont focalisés sur la détection IPA et ont cherché à concevoir une méthode permettant d'exploiter des spectres neutroniques issus de réactions photo-nucléaires où plusieurs énergies de photons sont en jeu. En d'autres termes, les inventeurs ont cherché à exploiter les spectres de neutrons obtenus par irradiation à partir de photons de bremsstrahlung ou à plusieurs raies (donc au moins deux raies).

### EXPOSÉ DE L'INVENTION

Ce but est notamment atteint grâce à l'utilisation d'un réseau de neurones entrainé.

L'invention concerne ainsi un procédé pour extraire des composantes neutroniques discrètes, issues de réactions photo-nucléaires entre des photons et au moins un élément chimique à détecter, d'un spectre neutronique issu de réactions photo-nucléaires obtenues par irradiation, d'un matériau comprenant ledit au moins un élément chimique à détecter, avec une source de photons, au moins une énergie des photons de la source étant supérieure au seuil de réaction photo-nucléaire dudit élément chimique à détecter, en utilisant un réseau de neurones multicouche et multicanal ayant une architecture avec un étage de convolution et un étage de déconvolution ;
dans lequel, si ledit au moins un élément chimique à détecter est choisi parmi le carbone, l'azote, l'oxygène ou le chlore et si la source de photons est une première source de photons à plusieurs énergies, le procédé comprend :
   - une étape préalable d'entrainement du réseau de neurones par apprentissage supervisé, qui s'effectue par itération à partir :
      ▪ de premiers spectres neutroniques, issus de réactions photo-nucléaires obtenues par irradiation du matériau avec des photons de bremsstrahlung; et
      ▪ de deuxièmes spectres neutroniques, chacun des deuxièmes spectres neutroniques étant issus de réactions photo-nucléaires induites par irradiation du matériau avec des photons d'une même énergie, qui est supérieure au seuil de réaction photo-nucléaire de l'élément chimique à détecter, et inférieure ou égale à l'énergie maximale des photons de ladite première source de photons à plusieurs énergies,
les premiers et deuxièmes spectres étant des spectres fictifs établis par une simulation Monte-Carlo, les premiers spectres étant fournis en entrée du réseau de neurones, et les deuxièmes spectres servant au calcul d'une fonction de coût destinée à ajuster des poids et des biais du réseau de neurones ; et
   - une étape de prédiction, à l'aide du réseau de neurones entrainé, comprenant :
      ▪ la fourniture, en entrée du réseau de neurones entrainé, du spectre neutronique issu de réactions photo-nucléaires induites par irradiation du matériau par ladite première source de photons ; et
      ▪ si le matériau irradié comporte, dans sa composition, au moins un élément chimique choisi parmi le carbone, l'azote, l'oxygène ou le chlore, l'obtention, en sortie du réseau de neurones entrainé et pour au moins une énergie spécifique d'intérêt de ladite première source de photons, d'un spectre neutronique prédit, comportant des composantes neutroniques discrètes issues de réactions photo-nucléaires dudit élément chimique choisi avec ladite énergie spécifique d'intérêt ; ou
dans lequel, si le matériau à irradier comprend ledit au moins un élément chimique à détecter choisi parmi le carbone, l'azote, l'oxygène ou le chlore, et au moins un autre élément chimique différent, apte à émettre des neutrons et qui n'est pas du carbone, de l'azote, de l'oxygène ou du chlore, et si la source de photons est une deuxième source de photons à une seule énergie ou à plusieurs énergies (à raies ou de Bremsstrahlung), le procédé comprend :
   - une étape préalable d'entrainement du réseau de neurones par apprentissage supervisé, qui s'effectue par itération à partir :
      ▪ de premiers spectres neutroniques, issus de réactions photo-nucléaires obtenues par irradiation du matériau avec des photons de bremsstrahlung, à raies ou mono-énergie ; et
      ▪ de deuxièmes spectres neutroniques, chacun des deuxièmes spectres neutroniques étant issus de réactions photo-nucléaires induites par irradiation du matériau avec des photons d'énergie supérieure au seuil de réaction photo-nucléaire dudit au moins un élément chimique à détecter et supérieure au seuil de réaction photo-nucléaire dudit autre élément chimique différent ;
les premiers et deuxièmes spectres étant des spectres fictifs établis par une simulation Monte-Carlo, les premiers spectres étant fournis en entrée du réseau de neurones, et les deuxièmes spectres servant au calcul d'une fonction de coût destinée à ajuster des poids et des biais du réseau de neurones ; et
   - une étape de prédiction, à l'aide du réseau de neurones entrainé, comprenant :
      ▪ la fourniture, en entrée du réseau de neurones entrainé, du spectre neutronique issu de réactions photo-nucléaires induites par irradiation du matériau par ladite deuxième source de photons ; et
      ▪ si le matériau irradié comporte, dans sa composition, au moins un élément chimique choisi parmi le carbone, l'azote, l'oxygène ou le chlore, l'obtention, en sortie du réseau de neurones entrainé et pour au moins une énergie spécifique d'intérêt de ladite deuxième source de photons, d'un spectre neutronique prédit, comportant des composantes neutroniques discrètes issues de réactions photo-nucléaires dudit élément chimique à détecter avec ladite énergie spécifique d'intérêt.

Dans le premier cas où le au moins un élément chimique à détecter est choisi parmi le carbone, l'azote, l'oxygène ou le chlore et si la source de photons est une première source de photons à plusieurs énergies (ci-après désigné sous le terme « premier cas »), alors on peut extraire, d'un spectre neutronique produit par une source de photons bremsstrahlung ou de photons à raies, des composantes neutroniques discrètes, comme si le spectre neutronique avait été produit par irradiation avec une source de photons mono-énergie (c'est-à-dire d'une seule et même énergie ou mono-énergétique).

Dans le deuxième cas où le matériau à irradier comprend ledit au moins un élément chimique à détecter choisi parmi le carbone, l'azote, l'oxygène ou le chlore, et au moins un autre élément chimique différent, apte à émettre des neutrons et qui n'est pas du carbone, de l'azote, de l'oxygène ou du chlore, et si la source de photons est une deuxième source de photons à une seule énergie ou à plusieurs énergies (à raies ou de Bremsstrahlung) (ci-après désigné sous le terme « deuxième cas »), on peut extraire, d'un spectre neutronique produit par irradiation d'un ensemble complexe (matériau comprenant au moins un élément léger et objet dans lequel il se trouve) à l'aide d'une source de photons (que les photons soient de bremsstrahlung, à plusieurs raies ou mono-énergie), des composantes neutroniques discrètes créés par ledit au moins un élément léger.

Dans ces deux cas de figures, seule l'étape d'entrainement est différente. Les spectres fournis pour le calcul de la fonction de coût sont des spectres neutroniques issus de d'irradiation mono-énergétiques dans le premier cas, et des spectres issus d'irradiation sur des éléments uniques dans le deuxième cas. On entend par « élément unique » le fait que le matériau irradié est constitué d'un seul élément (ou, en d'autres termes, un élément pur), c'est-à-dire que, dans le matériau, l'élément n'est pas mélangé à d'autres éléments différents.

À titre d'illustration, dans le deuxième cas, lors de l'étape d'entrainement, le réseau de neurones se voit fournir des spectres neutroniques issus d'irradiation avec des photons de Bremsstrahlung d'échantillons ou de substances diverses en entrée de réseau. L'apprentissage, pour les réseaux de neurones, consiste à calculer des paramètres de telle manière que les sorties du réseau de neurones soient, pour les exemples utilisés lors de l'apprentissage, aussi proches que possible des sorties « désirées » (ici : des spectres neutroniques issus d'irradiation avec des photons monoénergétiques). Pendant l'apprentissage, on cherche donc à minimiser l'écart entre les réponses réelles du réseau et les réponses « désirées », en modifiant les paramètres par étapes successives. On va alors « montrer » au réseau ces réponses « désirées » pour chaque spectre neutronique issu d'irradiation avec des photons de Bremsstrahlung d'échantillons ou de substances diverses, et fourni en entrée de réseau. Ces réponses « désirées » ne constituent pas réellement les données d'entrée à proprement parler, comme le sont les spectres neutroniques issus d'irradiation avec des photons de Bremsstrahlung.

Selon une variante avantageuse de l'invention, l'énergie spécifique d'intérêt est supérieure ou égale à 17 MeV.

En fait, dans l'étape de prédiction, on peut obtenir un spectre neutronique pour une énergie spécifique d'intérêt ou bien plusieurs spectres, pour deux ou plusieurs énergies différentes de la source. Cependant, afin de restreindre le coût en calculs, on préfère prédire un spectre neutronique pour une seule énergie de photons de la source, choisie en fonction de l'élément chimique à détecter. Dans le cadre de l'application à la détection d'explosifs où l'on s'intéresse à la détection de l'azote, le choix se porte sur l'énergie de 17 MeV, qui est la plus appropriée pour la détection de l'azote.

L'invention concerne également un procédé de détection d'un élément chimique à détecter choisi parmi le carbone, l'azote, l'oxygène ou le chlore, le procédé comprenant :
- l'irradiation, par une source de photons à plusieurs énergies (les photons peuvent donc être de bremsstrahlung ou à raies), d'un matériau comprenant ledit élément chimique ;
- la détection des neutrons émis par le matériau irradié et l'acquisition d'un spectre neutronique correspondant ;
- l'extraction, du spectre neutronique acquis, des composantes neutroniques discrètes dues audit élément chimique à détecter, par mise en œuvre du procédé pour extraire des composantes neutroniques discrètes tel qu'exposé ci-dessus, dans le cas où la source de photons est la première source ;
- comparaison des composantes neutroniques discrètes extraites avec une bibliothèque de composantes neutroniques discrètes associées au carbone, à l'azote, à l'oxygène et au chlore, et identification d'une correspondance, moyennant quoi on en déduit la présence dudit élément chimique.

L'invention concerne enfin un procédé de détection d'un matériau à détecter contenu dans un objet à sonder, le matériau à détecter comprenant au moins un élément chimique à détecter choisi parmi le carbone, l'azote, l'oxygène ou le chlore, et l'objet à sonder comprenant au moins un autre élément chimique différent, apte à émettre des neutrons et qui n'est pas du carbone, de l'azote, de l'oxygène ou du chlore, le procédé comprenant :
- l'irradiation, par une source de photons (qui peut être une source de photons de Bremsstrahlung, à raies ou à mono-énergie), de l'objet à sonder et du matériau à détecter qu'il contient ;
- la détection des neutrons émis par l'objet à sonder et le matériau à détecter et l'acquisition d'un spectre neutronique correspondant ;
- l'extraction, du spectre neutronique acquis, des composantes neutroniques discrètes dues audit au moins un élément chimique, par mise en œuvre du procédé pour extraire des composantes neutroniques discrètes tel qu'exposé ci-dessus dans le cas où la source de photons est la deuxième source ;
- comparaison des composantes neutroniques discrètes extraites avec une bibliothèque de composantes neutroniques discrètes associées à des matériaux comprenant ledit au moins un premier élément chimique, et identification d'une correspondance, moyennant quoi on en déduit la présence dudit matériau à détecter.

Dans les différents aspects de l'invention, l'utilisation d'un réseau de neurones permet de s'affranchir du besoin d'utiliser une source mono-énergétique au profit de l'utilisation d'une source plus conventionnelle, plus intense et facilement accessible comme, par exemple, une source basée sur un accélérateur linéaire d'électrons générant un spectre en énergie continu de photons (rayonnement de bremsstralhung) qui ont l'avantage d'être fortement orientés vers l'avant (donnant une source directionnelle).

Dans le cadre de l'invention, on parle de composantes neutroniques qui sont qualifiées de « discrètes ». Cela s'entend dans le sens où les composantes neutroniques forment des structures en pics plus ou moins larges, qui sortent clairement du continuum des spectres neutroniques. La largeur à mi-hauteur de ces pics dépendra notamment de la résolution en énergie des spectres.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
- la figure 1 représente, de manière schématique, un montage pour illustrer un mode de réalisation de l'invention ;
- les figures 2a à 2c représentent respectivement un spectre de photons bremsstrahlung, tel qu'obtenu après la cible 2 de la figure 1, un spectre des neutrons produits par l'irradiation d'un matériau explosif avec des photons de bremsstrahlung (figure 2b), tel qu'obtenu par le détecteur 5 de la figure 1, et un spectre des neutrons prédit pour une irradiation de ce même matériau explosif avec des photons de 17 MeV (figure 2c), tel qu'obtenu à la sortie d'un réseau de neurones entrainé 6 selon l'invention, avec une identification des éléments légers présents dans sa composition ;
- les figures 3a et 3b représentent, de manière schématique, un réseau de neurones multicouche et multicanal (figure 3a) et un diagramme pour décrire une architecture de ce réseau de neurones, avec un étage de convolution et un étage de déconvolution (figure 3b) ;
- la figure 4 représente un triangle des proportions normalisées en azote, carbone et oxygène de plusieurs substances ou produits, avec une identification des groupes entrant dans la catégorie des matières illicites.

### EXPOSÉ DÉTAILLÉ DE MODES DE RÉALISATION PARTICULIERS

Selon un premier aspect de l'invention, le procédé selon l'invention permet d'extraire des composantes neutroniques issues de réactions photo-nucléaires, pour chaque énergie de photons composant une source dont les photons ont un spectre en énergie continu (bremsstrahlung) ou à plusieurs énergies (à raies). Le procédé selon l'invention permet de remplacer la méthode existante dite du photon « taggé » par l'utilisation d'un réseau de neurones multicouche et multicanal, ce qui permet de s'affranchir du besoin en électronique spécialisée et en dipôles magnétiques que nécessite la méthode du photon « taggé ». Le procédé selon l'invention permet également de s'affranchir du besoin d'utiliser une source mono-énergétique. L'obtention de spectres neutroniques pour chaque énergie de photons est ici réalisée en post-traitement, sans ressource physique supplémentaire que celles utilisées classiquement en détection IPA, limitant ainsi les coûts et l'encombrement du dispositif de détection des éléments légers utilisé.

Ainsi, l'utilisation d'un réseau d'apprentissage profond dit « réseau de neurones » selon l'invention permet de retrouver les contributions neutroniques dans le spectre total généré par les photons issus d'une source de bremsstrahlung ou à raies pour les différentes énergies des photons de la source. L'identification des éléments légers est ainsi rendue possible dans les différentes contributions neutroniques extraites.

Selon un deuxième aspect de l'invention, le procédé selon l'invention permet également d'extraire les composantes neutroniques dues à des éléments légers d'une matrice complexe (qui peut être le matériau dans lequel se trouvent le ou les éléments légers et/ou l'objet dans lequel ce matériau est situé).

Quels que soient les aspects de l'invention, la spécificité de l'approche proposée consiste en l'apprentissage et l'utilisation d'un réseau de neurones multicouche et multicanal entrainé de façon à pouvoir prédire les composantes neutroniques pour chaque énergie des photons de bremsstrahlung ou à raies, lorsque l'on lui fournit un spectre neutronique issu de l'irradiation de matériaux par des photons de bremsstrahlung ou à raies, ou lorsqu'on cherche à analyser des spectres neutroniques issus de tout type de source de photon (mono-énergétique, à raies ou de bremsstralhung) pour extraire les contributions neutroniques des éléments légers d'une matrice complexe.

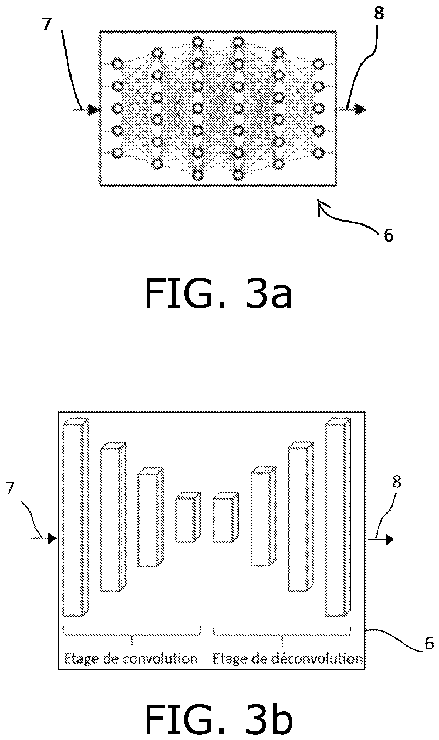

Le réseau de neurones 6 est multicouche et multicanal (figure 3a).

Il est multicouche, car il a une couche d'entrée, une couche de sortie et une ou plusieurs couches intermédiaires, dites couches cachées.

Il est multicanal, car chaque couche comporte plusieurs canaux. Chaque canal va représenter un intervalle en énergie du spectre. Chaque intervalle en énergie d'un spectre neutronique correspond à un nœud d'entrée 7 et à un nœud de sortie 8. Par exemple, si on veut retracer un spectre neutronique avec une énergie allant de 1 à 11 MeV, on peut avoir 100 canaux pour chaque énergie avec un incrément (pas d'échantillonnage) de 0,1 MeV. Plus le nombre de canaux est important, plus la valeur du pas d'échantillonnage est petite et plus on obtient une résolution précise.

Le réseau de neurones 6 dispose d'une architecture en deux parties. Il est en effet composé d'un étage de convolution et d'un étage de déconvolution (figure 3b).

Le premier étage permet l'extraction de caractéristiques et de motifs des spectres neutroniques introduits en entrée 7 du réseau 6 et permet une réduction des données. Le deuxième étage assure la reconstruction d'un spectre à partir des caractéristiques extraites par le premier étage, tout en retrouvant la dimension des données initiales.

De manière connue, l'apprentissage supervisé du réseau de neurones repose sur la série d'étapes suivantes, pour n couples de valeurs entrée-cible, n étant un nombre entier qui dépend de la résolution en énergie des spectres et correspond au nombre d'intervalles en énergie des spectres :
1- présentation au réseau d'un des couples « spectre entrée - spectre cible » (ci-après « spectres entrée-cible ») des n intervalles ;
2- calcul d'une prévision du réseau pour la cible attendue ;
3- utilisation d'une fonction de coût pour calculer la différence entre la prévision (sortie) du réseau et le spectre cible ;
4- utilisation de l'algorithme d'apprentissage du réseau de neurones pour ajuster les poids et les biais du réseau, de telle sorte que le réseau produise de meilleures prévisions à chaque présentation d'un couple de spectres entrée-cible.

Il est à noter que les étapes 1 à 4 constituent une seule itération (ou cycle d'apprentissage).

On répète les étapes 1 à 4 pendant un certain nombre d'itérations jusqu'à ce que le réseau commence à produire des résultats suffisamment fiables (c'est-à-dire des sorties qui se trouvent assez proches des cibles compte tenu des valeurs d'entrée). Le nombre d'itérations nécessaire pour entraîner le réseau de neurones n'est pas connu *a priori* mais est généralement supérieur à plusieurs centaines, voire millier de fois.

Dans le cas présent, pour chaque couple de spectres entrée-cible, l'entrée est un premier spectre fictif (par référence à la dénomination « premiers spectres neutroniques » utilisée pour définir l'invention), par exemple un spectre neutronique produit par des photons de bremsstrahlung et, pour la cible, un deuxième spectre fictif (par référence à la dénomination « deuxièmes spectres neutroniques » utilisée pour définir l'invention) produit par une source mono-énergie. Ainsi, à titre d'exemple, l'apprentissage supervisé du réseau de neurone s'effectue par itération à partir de spectres neutroniques issus de l'irradiation par des photons de bremsstrahlung (« premiers spectres fictifs ») qui sont fournis en entrée 7 du réseau et de spectres neutroniques produits par des photons à une seule énergie (« deuxièmes spectres fictifs »), qui servent au calcul d'une fonction de coût qui permettra l'ajustement des poids et des biais du réseau 6 pour que sa prédiction soit la plus proche possible des spectres issus de la déconvolution attendue.

Pour l'apprentissage, on utilise des spectres fictifs qui sont établis par simulation Monte-Carlo, ce qui permet d'obtenir facilement une multitude de données de couple entrée-cible. Des techniques d'augmentation de données sont appliquées pour multiplier les spectres en faisant varier faiblement et aléatoirement l'intensité de chaque canal afin d'avoir un nombre de spectres différents suffisant pour l'apprentissage du réseau. On peut par exemple utiliser le logiciel code de transport Monte-Carlo à N particules (MCNP^{™}), qui permet de modéliser des processus de physique nucléaire en utilisant la méthode de Monte-Carlo.

Une fois son apprentissage terminé, les poids et les biais du réseau ainsi ajusté sont enregistrés et le réseau de neurones peut être utilisé à des fins de prévision sans qu'un spectre cible ne lui soit fourni.

Par exemple, un spectre neutronique obtenu par irradiation avec une source de photons à plusieurs énergies est fourni en entrée du réseau entrainé, qui va le déconvoluer et en extraire numériquement les composantes neutroniques, pour une énergie spécifique d'intérêt (on peut aussi extraire les composantes neutroniques pour plusieurs énergies de la source, ou encore pour chaque énergie des photons de la source).

L'analyse des spectres neutroniques prédits obtenus en sortie du réseau de neurones permet, par exemple, de détecter dans un matériau irradié par des photons de bremsstrahlung, la présence d'éléments légers à partir de pics qui deviennent beaucoup plus facilement identifiables et dont ils sont la signature. Chaque pic dans le spectre prédit correspond à un niveau d'énergie d'un noyau résiduel produit par réaction (y,n), qui est donc un isotope du noyau initial. Les niveaux d'énergie qui sont propres à chaque noyau résiduel permettent d'identifier l'élément présent dans le matériau à détecter 4.

Pour illustrer l'invention, nous allons mettre en œuvre le procédé selon l'invention en utilisant une source de photons de bremsstrahlung pour détecter de la TNT (de formule C₇H₅N₃O₆) contenue dans un objet en bois (cellulose, de formule (C₆H₁₀O₅)n) en y induisant des réactions photonucléaires.

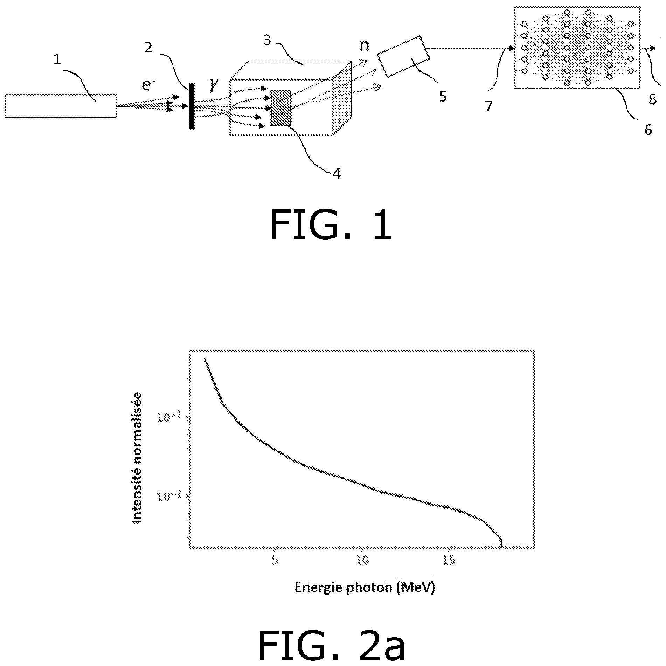

En se référant à la figure 1, un exemple d'un montage pour réaliser l'invention est illustré schématiquement. Un accélérateur linéaire d'électrons 1 génère un rayonnement d'électrons (e-) qui sont dirigés vers une cible 2 particulière (par exemple une cible mince en or), ce qui produit un rayonnement de photons (y) qui sont dirigés sur l'objet à sonder 3 et sur le matériau à détecter 4 qu'il contient. L'interaction des photons y avec le matériau à détecter 4 produit des neutrons, qui sont détectés par un détecteur 5 permettant de réaliser une spectrométrie des neutrons. Le détecteur 5 peut par exemple être choisi parmi des spectromètres à sphères de Bonner ou des scintillateurs.

Le spectre neutronique obtenu en sortie du détecteur 5 est introduit, en entrée 7, dans le réseau de neurones 6 entrainé, et on obtient, en sortie 8 de ce réseau de neurones, un spectre neutronique prédit dans lequel sont présentes les compositions neutroniques discrètes extraites.

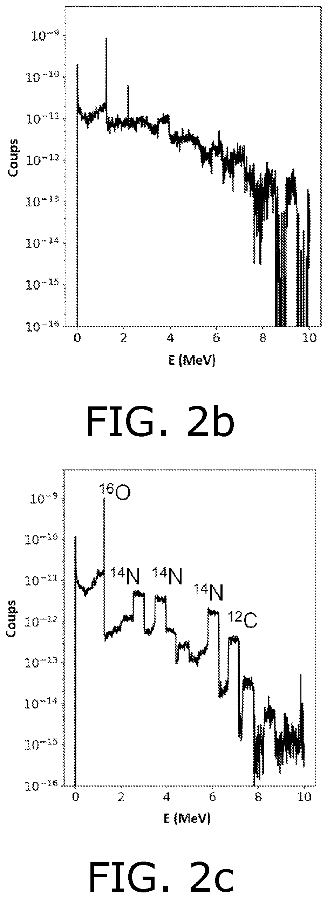

À titre d'exemple, l'objet à sonder 3 peut être un colis en bois ou en carton renfermant, comme matériau à détecter 4, une substance qui est un matériau explosif, par exemple de la TNT ; le spectre de photons de bremsstrahlung obtenu après la cible 2 est illustré dans la figure 2a ; le spectre neutronique obtenu en sortie du détecteur 5 par irradiation du matériau explosif avec les photons de bremsstrahlung est illustré dans la figure 2b ; le spectre neutronique prédit par le réseau de neurones 6 entrainé (et qui est obtenu à la sortie 8 du réseau de neurones 6) est illustré dans la figure 2c. Le spectre prédit est en fait identique à un spectre obtenu pour une irradiation du matériau explosif avec des photons de 17 MeV, qui est une énergie particulièrement intéressante dans le cadre de la détection d'azote (N) pour les matières illicites ; les différents pics (qui correspondent aux composantes neutroniques discrètes extraites par le procédé) ont été identifiés et attribués aux éléments légers ¹⁶O, ¹⁴N et ¹²C.

L'emplacement et la hauteur des pics forme une signature spécifique qui est comme une empreinte et permet d'identifier la présence de TNT. On est donc ainsi capable de déterminer que le colis contient de la TNT.

Dans cet exemple de mise en œuvre de l'invention, une source conventionnelle produisant des photons de bremsstrahlung est utilisée pour induire dans le matériau à sonder 3 des réactions photo-nucléaires, mais on aurait pu utiliser une source à mono-énergie ou à raies.

L'invention trouve application dans le domaine de la détection par la méthode d'interrogation photonique active (IPA) de matières illicites dans des containeurs et colis.

La détection d'éléments légers peut être réalisée sur la base des signatures spécifiques dans les spectres des neutrons extraits par le réseau de neurones.

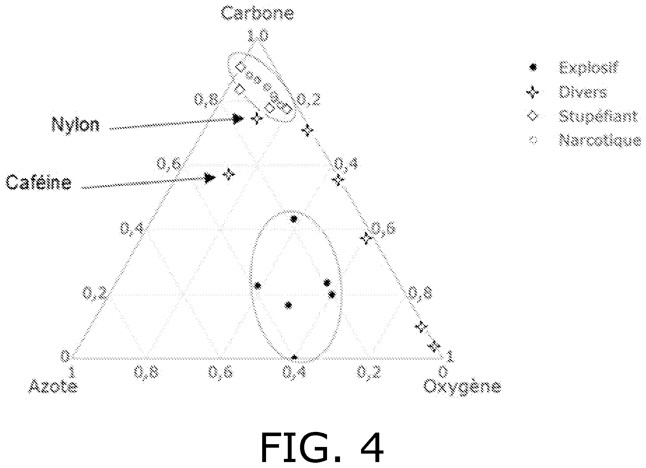

Il est également possible de déterminer les concentrations en éléments légers tels que l'azote, le carbone, l'oxygène ou encore le chlore à partir de la hauteur des composantes neutroniques discrètes de ces spectres neutroniques. Comme le montre la figure 4, la connaissance de la proportion de ces éléments légers (C, N, O, Cl) permet en effet de connaitre la nature du produit ou de la substance dissimulée dans l'objet sondé (colis, conteneur ou autre).

L'azote, par exemple, est présent dans la plupart des explosifs et pourra servir de marqueur pour détecter un matériau explosif de trinitrotoluène (TNT, de formule chimique C₇H₅O₆N₃), de cyclotriméthylènetrinitramine (RDX) ou tout autre explosif conventionnel, comme par exemple la nitroglycérine (C₃H₅O₉N₃), ou le C4 (C₄H₆O₆N₆).

On peut également détecter de la cocaïne (C₁₇H₂₁NO₄).

Quant aux gaz toxiques, leur présence peut être soupçonnée par la détection du chlore, présent par exemple dans le phosgène (COCl₂).

## Revendications

1. Procédé pour extraire des composantes neutroniques discrètes, issues de réactions photo-nucléaires entre des photons et au moins un élément chimique à détecter, d'un spectre neutronique issu de réactions photo-nucléaires obtenues par irradiation, d'un matériau comprenant ledit au moins un élément chimique à détecter, avec une source de photons, au moins une énergie des photons de la source étant supérieure au seuil de réaction photo-nucléaire dudit élément chimique à détecter, en utilisant un réseau de neurones multicouche et multicanal ayant une architecture avec un étage de convolution et un étage de déconvolution ;
dans lequel, si ledit au moins un élément chimique à détecter est choisi parmi le carbone, l'azote, l'oxygène ou le chlore et si la source de photons est une première source de photons à plusieurs énergies, le procédé comprend :
- une étape préalable d'entrainement du réseau de neurones par apprentissage supervisé, qui s'effectue par itération à partir :
▪ de premiers spectres neutroniques, issus de réactions photo-nucléaires obtenues par irradiation du matériau avec des photons de bremsstrahlung; et
▪ de deuxièmes spectres neutroniques, chacun des deuxièmes spectres neutroniques étant issus de réactions photo-nucléaires induites par irradiation du matériau avec des photons d'une même énergie, qui est supérieure au seuil de réaction photo-nucléaire de l'élément chimique à détecter, et inférieure ou égale à l'énergie maximale des photons de ladite première source de photons à plusieurs énergies,
les premiers et deuxièmes spectres étant des spectres fictifs établis par une simulation Monte-Carlo, les premiers spectres étant fournis en entrée du réseau de neurones, et les deuxièmes spectres servant au calcul d'une fonction de coût destinée à ajuster des poids et des biais du réseau de neurones ; et
- une étape de prédiction, à l'aide du réseau de neurones entrainé, comprenant :
▪ la fourniture, en entrée du réseau de neurones entrainé, du spectre neutronique issu de réactions photo-nucléaires induites par irradiation du matériau par ladite première source de photons ; et
▪ si le matériau irradié comporte, dans sa composition, au moins un élément chimique choisi parmi le carbone, l'azote, l'oxygène ou le chlore, l'obtention, en sortie du réseau de neurones entrainé et pour au moins une énergie spécifique d'intérêt de ladite première source de photons, d'un spectre neutronique prédit, comportant des composantes neutroniques discrètes issues de réactions photo-nucléaires dudit élément chimique choisi avec ladite énergie spécifique d'intérêt ; ou
dans lequel, si le matériau à irradier comprend ledit au moins un élément chimique à détecter choisi parmi le carbone, l'azote, l'oxygène ou le chlore, et au moins un autre élément chimique différent, apte à émettre des neutrons et qui n'est pas du carbone, de l'azote, de l'oxygène ou du chlore, et si la source de photons est une deuxième source de photons, à une seule énergie ou à plusieurs énergies, le procédé comprend :
- une étape préalable d'entrainement du réseau de neurones par apprentissage supervisé, qui s'effectue par itération à partir :
▪ de premiers spectres neutroniques, issus de réactions photo-nucléaires obtenues par irradiation du matériau avec des photons de bremsstrahlung, à raies ou mono-énergie ; et
▪ de deuxièmes spectres neutroniques, chacun des deuxièmes spectres neutroniques étant issus de réactions photo-nucléaires induites par irradiation du matériau avec des photons d'énergie supérieure au seuil de réaction photo-nucléaire dudit au moins un élément chimique à détecter et supérieure au seuil de réaction photo-nucléaire dudit autre élément chimique différent ;
les premiers et deuxièmes spectres étant des spectres fictifs établis par une simulation Monte-Carlo, les premiers spectres étant fournis en entrée du réseau de neurones, et les deuxièmes spectres servant au calcul d'une fonction de coût destinée à ajuster des poids et des biais du réseau de neurones ; et
- une étape de prédiction, à l'aide du réseau de neurones entrainé, comprenant :
▪ la fourniture, en entrée du réseau de neurones entrainé, du spectre neutronique issu de réactions photo-nucléaires induites par irradiation du matériau par ladite seconde source de photons ; et
▪ si le matériau irradié comporte, dans sa composition, au moins un élément chimique choisi parmi le carbone, l'azote, l'oxygène ou le chlore, l'obtention, en sortie du réseau de neurones entrainé et pour au moins une énergie spécifique d'intérêt de ladite deuxième source de photons, d'un spectre neutronique prédit, comportant des composantes neutroniques discrètes issues de réactions photo-nucléaires dudit élément chimique à détecter avec ladite énergie spécifique d'intérêt.

2. Procédé selon la revendication 1, dans lequel l'énergie spécifique d'intérêt est supérieure ou égale à 17 MeV.

3. Procédé de détection d'un élément chimique à détecter choisi parmi le carbone, l'azote, l'oxygène ou le chlore, le procédé comprenant :
- l'irradiation, par une source de photons à plusieurs énergies, d'un matériau comprenant ledit élément chimique ;
- la détection des neutrons émis par le matériau irradié et l'acquisition d'un spectre neutronique correspondant ;
- l'extraction, du spectre neutronique acquis, des composantes neutroniques discrètes dues audit élément chimique à détecter, par mise en œuvre du procédé selon la revendication 1, la source de photons étant la première source ;
- comparaison des composantes neutroniques discrètes extraites avec une bibliothèque de composantes neutroniques discrètes associées au carbone, à l'azote, à l'oxygène et au chlore, et identification d'une correspondance, moyennant quoi on en déduit la présence dudit élément chimique.

4. Procédé de détection d'un matériau à détecter contenu dans un objet à sonder, le matériau à détecter comprenant au moins un élément chimique à détecter choisi parmi le carbone, l'azote, l'oxygène ou le chlore, et l'objet à sonder comprenant au moins un autre élément chimique différent, apte à émettre des neutrons et qui n'est pas du carbone, de l'azote, de l'oxygène ou du chlore, le procédé comprenant :
- l'irradiation, par une source de photons, de l'objet à sonder et du matériau à détecter qu'il contient ;
- la détection des neutrons émis par l'objet à sonder et le matériau à détecter et l'acquisition d'un spectre neutronique correspondant ;
- l'extraction, du spectre neutronique acquis, des composantes neutroniques discrètes dues audit au moins un élément chimique, par mise en œuvre du procédé selon la revendication 1, la source de photons étant la deuxième source ;
- comparaison des composantes neutroniques discrètes extraites avec une bibliothèque de composantes neutroniques discrètes associées à des matériaux comprenant ledit au moins un premier élément chimique, et identification d'une correspondance, moyennant quoi on en déduit la présence dudit matériau à détecter.

## Patentansprüche

1. Verfahren zum Extrahieren diskreter Neutronenkomponenten, die aus photonuklearen Reaktionen zwischen Photonen und mindestens einem zu detektierenden chemischen Element resultieren, aus einem Neutronenspektrum, das aus photonuklearen Reaktionen resultiert, die durch Bestrahlung eines Materials, das das mindestens eine zu detektierende chemische Element umfasst, mit einer Photonenquelle erhalten werden, wobei mindestens eine Energie der Photonen der Quelle höher ist als der Schwellenwert der photonuklearen Reaktion des zu detektierenden chemischen Elements, unter Verwendung eines mehrschichtigen und mehrkanaligen Neuronennetzwerks mit einer Architektur mit einer Konvolutionsstufe und einer Dekonvolutionsstufe;
wobei, wenn das mindestens eine zu detektierende chemische Element aus Kohlenstoff, Stickstoff, Sauerstoff oder Chlor ausgewählt ist und wenn die Photonenquelle eine erste Mehrenergie-Photonenquelle ist, das Verfahren Folgendes umfasst:
- einen vorausgehenden Schritt zum Trainieren des Neuronennetzwerks durch überwachtes Lernen, der iterativ erfolgt, ausgehend von:
▪ ersten Neutronenspektren, die aus photonuklearen Reaktionen resultieren, die durch Bestrahlung des Materials mit Bremsstrahlungsphotonen erhalten werden; und
▪ zweiten Neutronenspektren, wobei jedes der zweiten Neutronenspektren aus photonuklearen Reaktionen resultiert, die durch Bestrahlung des Materials mit Photonen derselben Energie induziert werden, die über dem Schwellenwert der photonuklearen Reaktion des zu detektierenden chemischen Elements liegt, und kleiner oder gleich der maximalen Energie der Photonen der ersten Mehrenergie-Photonenquelle ist,
wobei die ersten und zweiten Spektren fiktive Spektren sind, die durch eine Monte-Carlo-Simulation erstellt wurden, wobei die ersten Spektren am Eingang des Neuronennetzwerks bereitgestellt werden, und die zweiten Spektren zur Berechnung einer Kostenfunktion dienen, die dazu bestimmt ist, Gewichte und Verzerrungen des Neuronennetzwerks anzupassen; und
- einen Schritt des Vorhersagens unter Verwendung des trainierten Neuronennetzwerks, umfassend:
▪ Bereitstellen, am Eingang des trainierten Neuronennetzwerks, des Neutronenspektrums, das aus photonuklearen Reaktionen resultiert, die durch Bestrahlung des Materials durch die erste Photonenquelle induziert werden; und
▪ wenn das bestrahlte Material in seiner Zusammensetzung mindestens ein chemisches Element aufweist, das aus Kohlenstoff, Stickstoff, Sauerstoff oder Chlor ausgewählt ist, Erhalten, am Ausgang des trainierten Neuronennetzwerks und für mindestens eine spezifische Energie von Interesse der ersten Photonenquelle, eines vorhergesagten Neutronenspektrums, das diskrete Neutronenkomponenten aufweist, die aus photonuklearen Reaktionen des ausgewählten chemischen Elements mit der spezifischen Energie von Interesse resultieren; oder
wobei, wenn das zu bestrahlende Material das mindestens eine zu detektierende chemische Element, das aus Kohlenstoff, Stickstoff, Sauerstoff oder Chlor ausgewählt ist, und mindestens ein weiteres, davon verschiedenes chemisches Element umfasst, das in der Lage ist, Neutronen zu emittieren und kein Kohlenstoff, Stickstoff, Sauerstoff oder Chlor ist, und wenn die Photonenquelle eine zweite Photonenquelle mit einer einzigen Energie oder mehreren Energien ist, das Verfahren Folgendes umfasst:
- einen vorausgehenden Schritt zum Trainieren des Neuronennetzwerks durch überwachtes Lernen, der iterativ erfolgt, ausgehend von:
▪ ersten Neutronenspektren, die aus photonuklearen Reaktionen resultieren, die durch Bestrahlung des Materials mit Bremsstrahlungs-, Linien- oder Monoenergiephotonen erhalten werden; und
▪ zweiten Neutronenspektren, wobei jedes der zweiten Neutronenspektren aus durch Bestrahlung des Materials mit Photonen induzierten photonuklearen Reaktionen mit Energie über dem Schwellenwert der photonuklearen Reaktion des mindestens einen zu detektierenden chemischen Elements und über dem Schwellenwert der photonuklearen Reaktion des anderen, davon verschiedenen chemischen Elements resultiert;
wobei die ersten und zweiten Spektren fiktive Spektren sind, die durch eine Monte-Carlo-Simulation erstellt wurden, wobei die ersten Spektren am Eingang des Neuronennetzwerks bereitgestellt werden, und die zweiten Spektren zur Berechnung einer Kostenfunktion dienen, die dazu bestimmt ist, Gewichte und Verzerrungen des Neuronennetzwerks anzupassen; und
- einen Schritt des Vorhersagens unter Verwendung des trainierten Neuronennetzwerks, umfassend:
▪ Bereitstellen, am Eingang des trainierten Neuronennetzwerks, des Neutronenspektrums, das aus photonuklearen Reaktionen resultiert, die durch Bestrahlung des Materials durch die zweite Photonenquelle induziert werden; und
▪ wenn das bestrahlte Material in seiner Zusammensetzung mindestens ein chemisches Element aufweist, das aus Kohlenstoff, Stickstoff, Sauerstoff oder Chlor ausgewählt ist, Erhalten, am Ausgang des trainierten Neuronennetzwerks und für mindestens eine spezifische Energie von Interesse der zweiten Photonenquelle, eines vorhergesagten Neutronenspektrums, das diskrete Neutronenkomponenten aufweist, die aus photonuklearen Reaktionen des zu detektierenden ausgewählten chemischen Elements mit der spezifischen Energie von Interesse resultieren.

2. Verfahren nach Anspruch 1, wobei die spezifische Energie von Interesse größer oder gleich 17 MeV ist.

3. Verfahren zum Detektieren eines zu detektierenden chemischen Elements, das aus Kohlenstoff, Stickstoff, Sauerstoff oder Chlor ausgewählt ist, das Verfahren umfassend:
- Bestrahlen eines Materials, das das chemische Element umfasst, durch eine Mehrenergie-Photonenquelle;
- Detektieren der vom bestrahlten Material emittierten Neutronen und Erfassen eines entsprechenden Neutronenspektrums;
- Extrahieren der diskreten Neutronenkomponenten aus dem erfassten Neutronenspektrum aufgrund dieses zu detektierenden chemischen Elements durch das Durchführen des Verfahrens nach Anspruch 1, wobei die Photonenquelle die erste Quelle ist;
- Vergleichen der extrahierten diskreten Neutronenkomponenten mit einer Bibliothek diskreter Neutronenkomponenten in Verbindung mit Kohlenstoff, Stickstoff, Sauerstoff und Chlor und Identifizieren einer Übereinstimmung, wodurch das Vorhandensein des chemischen Elements abgeleitet wird.

4. Verfahren zum Detektieren eines in einem zu messenden Objekt enthaltenen zu detektierenden Materials, wobei das zu detektierende Material mindestens ein zu detektierendes chemisches Element umfasst, das aus Kohlenstoff, Stickstoff, Sauerstoff oder Chlor ausgewählt ist, und das zu messende Objekt mindestens ein anderes chemisches Element umfasst, das geeignet ist, Neutronen zu emittieren, und das kein Kohlenstoff, Stickstoff, Sauerstoff oder Chlor ist, wobei das Verfahren Folgendes umfasst:
- Bestrahlen des zu messenden Objekts und des darin enthaltenen zu detektierenden Materials durch eine Photonenquelle;
- Detektieren der vom zu messenden Objekt und dem zu detektierenden Material emittierten Neutronen und Erfassen eines entsprechenden Neutronenspektrums;
- Extrahieren der diskreten Neutronenkomponenten aus dem erfassten Neutronenspektrum aufgrund des mindestens einen chemischen Elements durch das Durchführen des Verfahrens nach Anspruch 1, wobei die Photonenquelle die zweite Quelle ist;
- Vergleichen der extrahierten diskreten Neutronenkomponenten mit einer Bibliothek diskreter Neutronenkomponenten in Verbindung mit Materialien, die das mindestens eine erste chemische Element umfassen, und Identifizieren einer Übereinstimmung, wodurch das Vorhandensein des zu detektierenden Materials abgeleitet wird.

## Claims

1. Method for extracting discrete neutron components, from photo-nuclear reactions between photons and at least one chemical element to be detected, from a neutron spectrum from photo-nuclear reactions obtained by irradiating a material comprising said at least one chemical element to be detected with a photon source, at least one energy of the photons of the source being greater than the photo-nuclear reaction threshold of said chemical element to be detected, using a multilayer and multichannel neural network having an architecture with a convolution stage and a deconvolution stage;
wherein, if said at least one chemical element to be detected is chosen from carbon, nitrogen, oxygen or chlorine and if the photon source is a first multi-energy photon source, the method comprises:
- a preliminary step of training the neural network by supervised learning, which is performed by iteration using:
▪ first neutron spectra, from photo-nuclear reactions obtained by irradiating the material with bremsstrahlung photons; and
▪ second neutron spectra, each of the second neutron spectra being from photo-nuclear reactions induced by irradiating the material with photons of the same energy, which is greater than the photo-nuclear reaction threshold of the chemical element to be detected, and less than or equal to the maximum energy of the photons of said first multi-energy photon source,
the first and second spectra being notional spectra established by a Monte-Carlo simulation, the first spectra being supplied at the input of the neural network, and the second spectra serving to compute a cost function intended to adjust the weights and biases of the neural network; and
- a prediction step, using the trained neural network, comprising:
▪ supplying, at the input of the trained neural network, the neutron spectrum from photo-nuclear reactions induced by irradiating the material with said first photon source; and
▪ if the irradiated material comprises, in its composition, at least one chemical element chosen from carbon, nitrogen, oxygen, or chlorine, obtaining, at the output of the trained neural network and for at least one specific energy of interest of said first photon source, a predicted neutron spectrum, comprising discrete neutron components from photo-nuclear reactions of said chosen chemical element with said specific energy of interest; or
wherein, if the material to be irradiated comprises said at least one chemical element to be detected chosen from carbon, nitrogen, oxygen or chlorine, and at least one other different chemical element, capable of emitting neutrons and which is not carbon, nitrogen, oxygen or chlorine, and if the photon source is a second, single-energy or multi-energy photon source, the method comprises:
- a preliminary step of training the neural network by supervised learning, which is performed by iteration using:
▪ first neutron spectra, from photo-nuclear reactions obtained by irradiating the material with bremsstrahlung, line or single-energy photons; and
▪ second neutron spectra, each of the second neutron spectra being from photo-nuclear reactions induced by irradiating the material with photons of an energy greater than the photo-nuclear reaction threshold of the chemical element to be detected and greater than the photo-nuclear reaction threshold of said other different chemical element;
the first and second spectra being notional spectra established by a Monte-Carlo simulation, the first spectra being supplied at the input of the neural network, and the second spectra serving to compute a cost function intended to adjust the weights and biases of the neural network; and
- a prediction step, using the trained neural network, comprising:
▪ supplying, at the input of the trained neural network, the neutron spectrum from photo-nuclear reactions induced by irradiating the material with said second photon source; and
▪ if the irradiated material comprises, in its composition, at least one chemical element chosen from carbon, nitrogen, oxygen or chlorine, obtaining, at the output of the trained neural network and for at least one specific energy of interest of said second photon source, a predicted neutron spectrum, comprising discrete neutron components from photo-nuclear reactions of said chemical element to be detected with said specific energy of interest.

2. Method according to claim 1, wherein the specific energy of interest is greater than or equal to 17 MeV.

3. Method for detecting a chemical element to be detected chosen from carbon, nitrogen, oxygen or chlorine, the method comprising:
- irradiating, with a multi-energy photon source, a material comprising said chemical element;
- detecting the neutrons emitted by the irradiated material and acquiring a corresponding neutron spectrum;
- extracting, from the neutron spectrum acquired, discrete neutron components due to said chemical element to be detected, by implementing the method according to claim 1, the photon source being the first source;
- comparing the discrete neutron components extracted with a library of discrete neutron components associated with carbon, nitrogen, oxygen and chlorine, and identifying a correlation, whereby the presence of said chemical element is inferred.

4. Method for detecting a material to be detected contained in an object to be probed, the material to be detected comprising at least one chemical element to be detected chosen from carbon, nitrogen, oxygen or chlorine, and the object to be probed comprising at least one other different chemical element, capable of emitting neurons and which is not carbon, nitrogen, oxygen or chlorine, the method comprising:
- irradiating, with a photon source, the object to be probed and the material to be detected contained therein;
- detecting the neutrons emitted by the object to be probed and the material to be detected and acquiring a corresponding neutron spectrum;
- extracting, from the neutron spectrum acquired, discrete neutron components due to said at least one chemical element to be detected, by implementing the method according to claim 1, the photon source being the second source;
- comparing the discrete neutron components extracted with a library of discrete neutron components associated with materials comprising said at least one first chemical element, and identifying a correlation, whereby the presence of said material to be detected is inferred.
